# EUROPÄISCHE PATENTSCHRIFT

(11) **EP 1 451 083 B1**
(45) Veröffentlichungstag und Bekanntmachung des Hinweises auf die Patenterteilung: **19.10.2005**
(21) Anmeldenummer: 02804177.0
(22) Anmeldetag: 16.11.2002
(51) Int. Cl.: B65D 83/08, A61F 15/00

(54) **SPENDEVORRICHTUNG FÜR FLÄCHENFÖRMIGE DARREICHUNGSFORMEN**
DISPENSING DEVICE FOR FLAT DOSAGE FORMS
DISPOSITIF DE DISTRIBUTION D'OBJETS DE FORME PLATE

(30) Priorität: 05.12.2001 DE 10159746
(43) Veröffentlichungstag der Anmeldung: 01.09.2004
(73) Patentinhaber: LTS LOHMANN Therapie-Systeme AG, 56626 Andernach (DE)
(72) Erfinder: VON FALKENHAUSEN, Christian, 53340 Meckenheim (DE); KRUMME, Markus, 56567 Neuwied (DE); GUTSCHE, Christian, 53340 Meckenheim (DE)
(74) Vertreter: Schmidt, Werner
(86) Internationale Anmeldenummer: PCT/EP2002/012874
(87) Internationale Veröffentlichungsnummer: WO 2003/048003

(56) Entgegenhaltungen:
- EP-A- 0 325 360
- WO-A-95/18046
- DE-A- 3 128 547
- US-A- 4 993 586
- US-A- 5 337 897
- US-A- 6 155 456
- US-B1- 6 213 343

## Beschreibung

Die Erfindung betrifft eine Vorrichtung zum Aufbewahren und Bereitstellen von gestapelten, mit folien- oder blattartigen Materialien befüllten Folientaschen. Die Folientaschen bestehen aus mindestens einer Trägerfolie und mindestens einer Deckfolie. Mindestens die flächenmäßig größere dieser Folien weist zwei parallele Seitenkanten auf. Zwischen der Trägerfolie und der Deckfolie ist das folien- oder blattartige Material gas- und feuchtedicht eingeschlossen. Die Trägerfolie steht an mindestens einer quer oder schräg zur Seitenkante liegenden Kante über die nächstliegende Kante der Deckfolie über. Die Vorrichtung umfasst ein mindestens einfach geteiltes Gehäuse, an dessen seitlichen Innenseiten die Seitenkanten der gestapelten Folientaschen anliegen, während an einer vorderen Innenseite diejenige Kante der Trägerfolie anliegt, die über die Kante der dazugehörenden Deckfolie übersteht. Dabei werden die gestapelten Folientaschen) federbelastet gegen die obere Innenseite des Gehäuses gepresst.

Aus der US 5,337,897 A ist ein derartiger Behälter bekannt. Die Folientaschen werden aus dem Behälter mittels einer Fingerkuppe herausgeschoben. Dazu hat das Gehäuse eine entsprechend große unverschließbare Öffnung. Auch muss der Nutzer die entnommene Folientasche separat öffnen, um das in der Folientasche integrierte folien- oder blattartigen Materialien nutzen zu können.

Ferner ist aus der WO 99/28211 A2 ein automatischer Blattspender z.B. für Visitenkarten bekannt. Der Blattspender besteht aus einem mit einem Deckel versehenen Behälter. Der Behälter ist mit einem Stapel einzelner Papierblätter befüllt. Der Stapel liegt über einen Träger auf einer Druckfeder auf, die ihn gegen eine im Deckel integrierte Transportwalze drückt. Im Bereich der Transportwalze befindet sich zwischen dem Deckel und dem Behälter ein Auswurfschlitz, durch den bei einer manuell eingeleiteten Rotation der Transportwalze Blätter bzw. Visitenkarten einzeln ausgeworfen werden.

Des Weiteren ist der US-DES 371,723 ist ein Behälter bekannt, der dem Aufbewahren und Bereitstellen von gestapelten mit folien- oder blattartigen Materialen, z.B. essbare Filme, dient. Der Filmstapel wird in den Behälter über eine große den Behälter ca. mittig trennenden Trennfuge befüllt. Das Bereitstellen der einzelnen Filme geschieht über eine separate, verschließbare Klappe. Die Filme sind direkt übereinander gestapelt und nur durch den Behälter mit den beiden nur bedingt dichten Trennfugen von der Umgebung getrennt. Neigen die essbaren Filme zum Aromaverlust oder zum Austrocknen, müssen sie schnell verbraucht werden, um einer Ungenießbarkeit vorzubeugen.

Der vorliegenden Erfindung liegt daher die Problemstellung zugrunde, eine Vorrichtung zum Aufbewahren und Bereitstellen von gestapelten folien- oder blattartigen Materialien zu entwickeln, die die gestapelten Materialien von der ersten bis zur letzten Entnahme ohne nennenswerte Änderung ihrer aromatischen Eigenschaften aufbewahrt.

Diese Problemstellung wird mit den Merkmalen des Hauptanspruches gelöst. Dazu hat der vordere, obere Bereich des Gehäuses zwei Schlitze für das getrennte Auswerfen der einzelnen Folientaschenteile, wobei das folien- oder blattartige Material mit einem der beiden Folientaschenteile ausgeworfen wird. Zwischen den Schlitzen ist ein Trennwerkzeug zum Trennen von Träger- und Deckfolie angeordnet, an dem die oberste Trägerfolie räumlich vor der Vorderkante der Deckfolie anliegt. Oberhalb der vorderen Hälfte der obersten Folientasche - auf dieser aufliegend - ist ein um eine Rotationsachse rotierbares Transportelement zumindest annähernd quer zu den Seitenkanten der Trägerfolie in einer Gehäuseöffnung angeordnet.

Mit dieser Erfindung werden die folien- oder blattartigen Materialien mindestens zweifach verpackt. Die Materialien sind beispielsweise filmartige Blättchen, deren Fläche ca. der eines mittleren Daumenabdrucks entspricht. Die Blättchen enthalten z.B. einen pharmazeutischen Wirkstoff. Der Verzehr der Blättchen erfolgt z.B. durch Auflegen auf die Zunge. Unter der Einwirkung von Speichel lösen sich die Blättchen in wenigen Sekunden auf. Der Wirkstoff wird hierbei freigesetzt.

Um die in den Blättchen enthaltenen, oft leichtflüchtigen Wirk- und/oder Aromastoffe bis zum Verzehr sicher zu erhalten, sind sie einzeln in dünnen Folientaschen z.B. eingesiegelt bzw. primärverpackt. Die in der Regel empfindlichen Folientaschen sind in gestapelter Form in einer sog. Sekundärverpackung geschätzt untergebracht. Die Sekundärverpackung ist hierbei ein Blättchenspender. Sie ist dazu mit einem Mechanismus ausgestattet, durch den die einzelne Folientasche ohne direkten Fingerkontakt direkt ins Freie gefördert wird. Bei diesem Vorgang wird die Folientasche automatisch geöffnet und das einzelne Blättchen zum Verzehr dargereicht.

Weitere Einzelheiten der Erfindung ergeben sich aus den Unteransprüchen und der nachfolgenden Beschreibung eines schematisch dargestellten Ausführungsbeispiels.
- Figur 1:: Folientasche im Längsschnitt;
- Figur 2:: Draufsicht zu Figur 2 ohne Deckfolie;
- Figur 3:: Sekundärverpackung im Längsschnitt;
- Figur 4:: Sekundärverpackung zu Figur 3 mit bereitgestellter Befüllung;
- Figur 5:: Sekundärverpackung zu Figur 3 mit letzter bereitgestellter Befüllung.

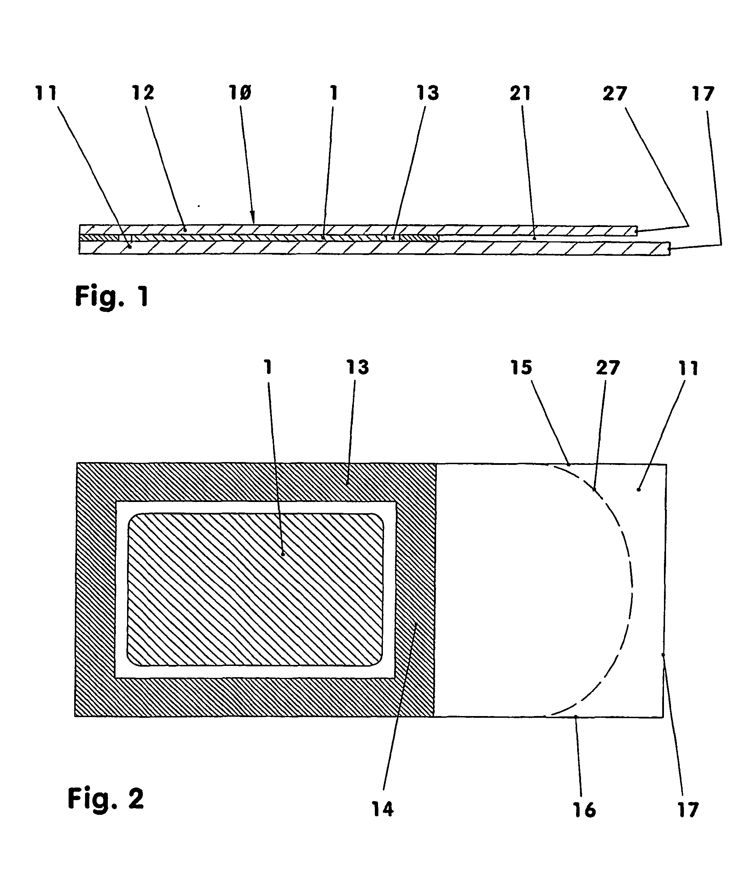

Die Figuren 1 und 2 zeigen eine Folientasche (10) mit einem eingelegten folien- oder blattartigen Material (1). Letzteres - auch Befüllung oder Blättchen genannt - ist beispielsweise ein Film, der einen pharmazeutischen Wirkstoff enthalten kann. Die Folientasche wird auch als Primärverpackung bezeichnet.

Die Folientasche besteht z.B. aus zwei Folien, zwischen denen der Film (1) vollflächig eingelegt ist. Um den Film (1) herum sind die beiden Folien (11, 12) in einem kleinen Abstand zu den Filmkanten verklebt, versiegelt, verschweigt oder dergleichen. Der Film (1) liegt folglich in einem gas- und feuchtdichten Hohlraum. Hierbei haftet er beispielsweise an der Deckfolie (12) stärker als an der Trägerfolie (11). Im Ausführungsbeispiel sind aus Vereinfachungsgründen die Folien (11, 12) beispielhaft mittels eines auftragenden Klebstoffes (13) miteinander verbunden.

Die Trägerfolie (11) ist eine rechteckige Folie, deren Länge z.B. dem Doppelten ihrer Breite entspricht. Auf der Trägerfolie (11) ist der Film (1) aufgelegt, vgl. Figur 1 und 2. Um den Film (1) herum ist die Trägerfolie (11) z.B. streifenförmig mit Klebstoff (13) bedruckt. Der Klebstoff (13) kommt hierbei nicht in Kontakt mit dem Film (1). Auf dem Film (1) und dem Klebstoff (13) ist die Deckfolie (12) - in Figur 2 gestrichelt dargestellt - festhaltend aufgelegt.

Die Deckfolie (12) hat hier die gleiche Breite wie die Trägerfolie (11). Allerdings ist sie kürzer. Ihre beispielsweise halbelliptische Vorderkante (27) ist nach hinten versetzt, z.B. um einige Millimeter. Zwischen der Vorderkante (27) und dem vorderen Abschnitt (14) des Klebstoffes (13) befindet sich auf ca. einem Drittel der Länge ein Spaltraum (21), vgl. Figur 1. In diesem Bereich haften die Folien (11, 12) nicht aneinander.

Alternativ zu der in den Figuren 1 - 4 dargestellten Folientasche (10) kann die Primärverpackung auch aus Folien bestehen, die im Kontaktbereich mit der Befüllung (1) eine Vertiefung aufweisen. Auch eine Vertiefung in nur einer Folie ist denkbar. In beiden Fällen liegen dann die Randbereiche der Folien trotz eingelegter Befüllung (1) vollflächig aufeinander auf, so dass die Höhe des Spaltraumes (21) nahezu unendlich klein wird.

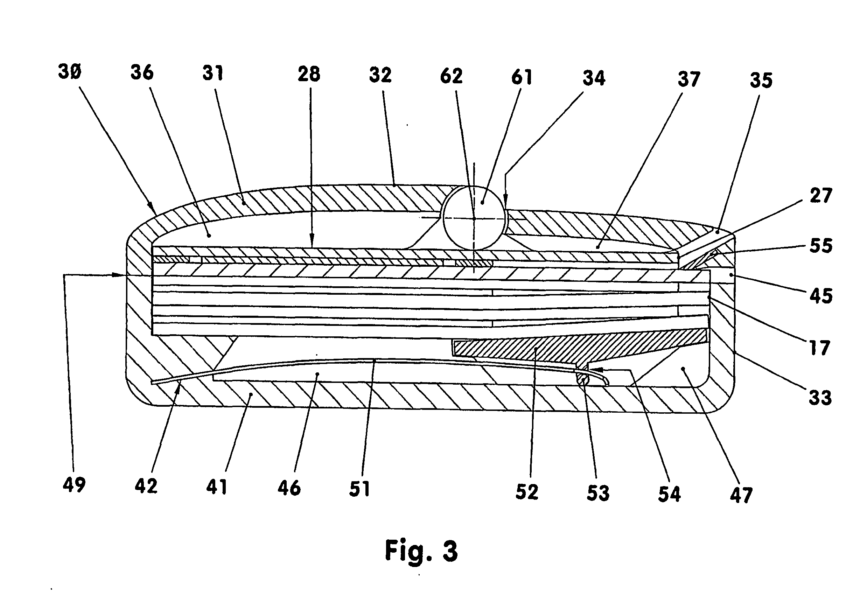

In den Figuren 3 - 5 ist eine sog. Sekundärverpackung (30) dargestellt, die die Folientaschen (10) zum einen aufbewahrt und zum anderen bei Bedarf bereitstellt. Bei dem Vorgang des Bereitstellens wird die Folientasche (10) zur Freigabe der Befüllung (1) geöffnet. Dies geschieht mit Hilfe eines in der Sekundärverpackung (30) angeordneten Trennwerkzeuges (55) und einer Transportwalze (61). Die Transportwalze (61) schiebt die Folientasche (10) über das Trennwerkzeug (55), das die Verbindungsfuge zwischen der Deckfolie (12) und der Trägerfolie (11) aufspaltet.
Um diesen Öffnungs- bzw. Spaltvorgang deutlich darstellen zu können, wird in den Figuren 1 - 5 die Folientasche (10) vereinfacht und schematisiert dargestellt. Die Stärken der Folien (11, 12) und des Klebers (13) sind im Verhältnis zu den anderen geometrischen Folienabmessungen erheblich vergrößert dargestellt.

Figur 3 zeigt als Sekundärverpackung (30) ein zweiteiliges Gehäuse (31, 41), in dem ein Stapel (28) aus Folientaschen (10) eingelegt ist. Die Folientaschen (10) liegen im Stapel (28) mit der Deckfolie (12) nach oben gleichsinnig orientiert übereinander. Nur die oberste Folientasche (10) ist in den Figuren 3 - 5 geschnitten dargestellt.

Das Gehäuse der Sekundärverpackung (30) umfasst hier beispielsweise ein wannenförmiges Unterteil (41) und ein deckelartiges Oberteil (31). Zwischen beiden Teilen (31, 41) befindet sich z.B. eine horizontale Trennfuge (49). Im Unterteil (41) ist eine Blattfeder (51) und eine wippenartige Andruckplatte (52) angeordnet. Die Blattfeder (51), sie ist über einen weiten Bereich zumindest annähernd parallel zum Stapel (28) orientiert, ist nach den Figuren 3 - 5 mit ihrem linken Ende im Unterteil (41) starr fixiert eingelassen. Am freien Ende der Blattfeder (51) ist die Andruckplatte (52) mit Spiel eingehängt. Dazu hat die Andruckplatte (52) einen z.B. weitgehend normal zur Blattfeder (51) ausgerichteten Arm (53), der einen Schlitz (54) zur Aufnahme der Blattfeder (51) aufweist. Die Blattfeder (51), die ohne Belastung durch den Stapel (28) nach oben federt, ist in dem Bereich zwischen der starren Einspannstelle (42) im Unterteil (41) und dem Schlitz (54) breiter ausgeführt als in dem Bereich, der durch den Schlitz (54) geführt ist. Dadurch wird verhindert, dass bei fast entleerter Sekundärverpackung (30) die Andruckplatte (52) unbeabsichtigt auf die Einspannstelle (42) zuwandert.

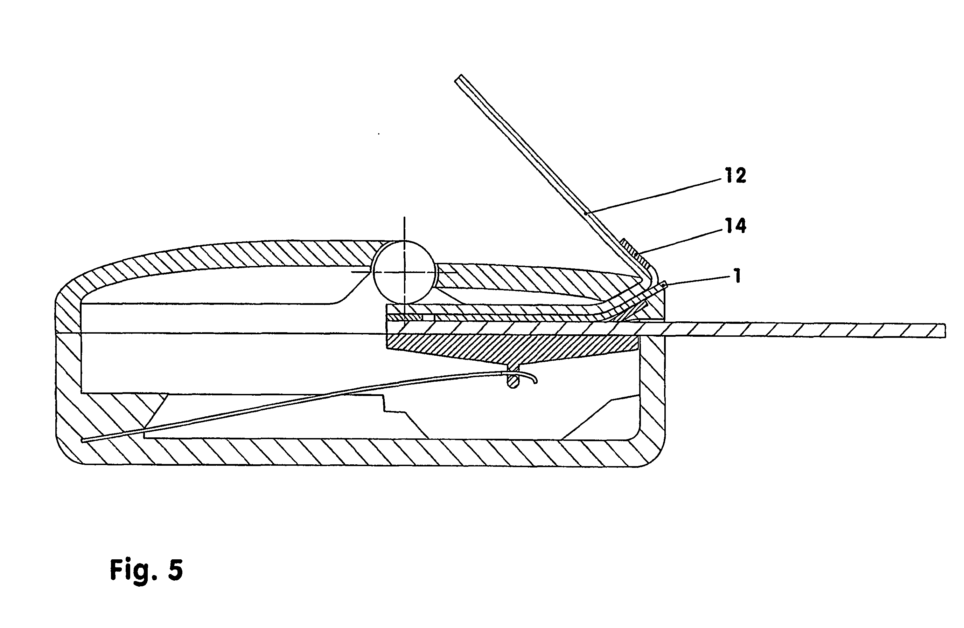

Das aus dem Schlitz (54) herausragende freie Ende der Blattfeder (51) ist nach unten gekröpft, um bei einem eventuellen Wiederbefüllen der Sekundärverpackung (30) ein Aushängen der Blattfeder (51) zu verhindern. Anstelle der Kröpfung kann an der Blattfeder (51) auch eine Öse bzw. ein Blattfederauge, eine Aufspaltung oder etwas Vergleichbares angeordnet sein. Ggf. kann die Blattfeder (51) als großflächiges Bauteil gestaltet sein, so dass sie auch die Funktion der dann wegfallenden Andruckplatte (52) übernehmen kann.
Im unteren Bereich des Unterteils (41) sind beidseits neben der Blattfeder (51) Stege (46, 47) oder Absätze z.B. ungeformt, die zumindest bei vollständig befüllter Sekundärverpackung (30) als Auflage für den Stapel (28) und die Andruckplatte (52) dienen, vgl. auch Figur 5.

Das Oberteil (31) sitzt in den Figuren 3 - 5 nur beispielhaft mittels einer ebenen Trennfuge (49) auf dem Unterteil (41). Beide Teile sind beispielsweise lösbar oder unlösbar miteinander verrastet, verklebt, verschraubt u.s.w., je nachdem, ob eine Wiederbefüllung der Sekundärverpackung (30) vorgesehen ist oder nicht.

Im Oberteil (31) ist in einer Gehäuseöffnung (34) etwa mittig die Transportwalze (61) angeordnet. Die Transportwalze (61) ist quer zur Längsausdehnung der Folientaschen (10) und zumindest annähernd parallel zur Oberfläche der obersten Folientasche (10) ausgerichtet. Im Ausführungsbeispiel sitzt die Transportwalze (61) im Bereich eines Gehäuseabsatzes. Dabei reicht der höher liegende Absatzbereich (32) zumindest annähernd tangential an die Transportwalze (61) heran, während der tiefer liegende Absatzbereich circa auf der Höhe der Walzenmitte endet. Durch diese Ausgestaltung ragt etwa ein Viertel des Transportwalzenumfangs aus der Kontur des Oberteils (31) heraus. Hierbei ist der Spalt zwischen der Transportwalze (61) und der Gehäuseöffnung (34) so eng, dass größere Schmutzpartikel nicht in das Gehäuseinnere gelangen können. Ggf. ist dieser Spalt zwischen der Transportwalze (61) und der Gehäuseöffnung (34) durch eine geeignete Dichtung abgedichtet.

Die Transportwalze (61) hat einen Durchmesser, der mindestens doppelt so groß ist wie die Wandstärke im Bereich der Gehäuseöffnung (34). Beispielsweise beträgt er mehrere Millimeter. An die Transportwalze (61) wird von unten her der Stapel (28) aufgrund der Federwirkung der Biegefeder (51) gedrückt. Als Material für die Transportwalze (61) wird ein Kunststoff oder eine Gummimischung verwendet, der mit dem Material der Deckfolienoberfläche eine Reibpaarung bildet, die eine hohe Haftreibungszahl sicherstellt.

Die Transportwalze (61) ist hier als ein glatter, zylindrischer Körper dargestellt. Die Zylinderoberfläche kann auch gerändelt oder anders profiliert sein. Z.B. kann die Profilierung eine Noppenstruktur sein. Selbstverständlich kann die Zylinderform auch nur eine ideelle Hüllfläche sein, die durch mehrere - nebeneinander auf einer Achse angeordnete - separate Rädchen vorgegeben wird.

Vor und hinter der Transportwalze (61) sind im Oberteil (31) Stege (36, 37) oder Gehäuseabsätze angeordnet. An letzteren liegt die jeweils obere Folientasche (10) an. Die Flächen, mit denen die Stege (36, 37) die Folientasche (10) kontaktieren, verlaufen zumindest bereichsweise tangential zur Oberfläche der Transportwalze (61). Die Stege (36, 37) gewährleisten eine flächige, tangentiale Anlage der obersten Folientasche (10) an der Transportwalze (61).

Das Gehäuse (31, 41) hat im Bereich seiner vorderen Stirnwand (33) zwei Schlitze (35) und (45). Beide Schlitze (35, 45) sind hier quer zur Längsausdehnung der Folientaschen (10) orientiert. Sie sind nur beispielsweise im Oberteil (31) integriert.

Der untere Schlitz (45) dient dem Auswurf der Trägerfolie (11). Er ist im Oberteil (31) unmittelbar vor dessen Vorderkante (17) platziert. Seine Breite und Höhe ist jeweils geringfügig größer als die jeweilige Breiten- und Stärkenabmessung der Trägerfolie (11).

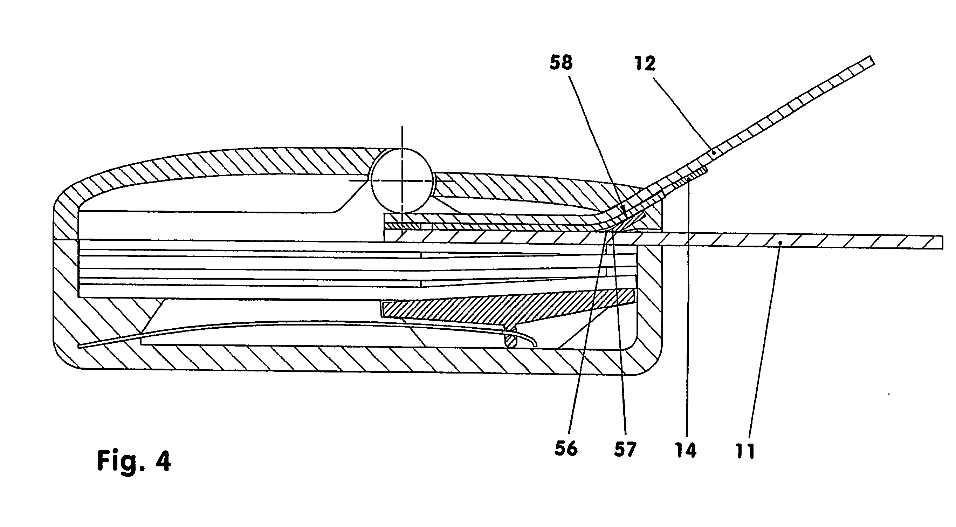

Oberhalb des unteren Schlitzes (45) ist im Oberteil (31) als Trennwerkzeug ein, z.B. ein gerades Messer (55), angeordnet. Das Messer (55) ist beispielsweise zur Befestigung im Oberteil (31) teilumspritzt oder eingelebt. Es ragt z.B. unter einem 30°-Win-kel gegenüber der Horizontalen bzw. der planen Oberfläche der Trägerfolie (11) in das Gehäuseinnere hinein. Die Einragtiefe ist so gewählt, dass die Schneidkante (56) des Messers (55), vgl. Figur 4, unmittelbar vor der vordersten Stelle der Vorderkante (27) der Deckfolie (12) endet. Die z.B. abgeschrägte Schneidflanke (57) des Messers (55) dient der obersten Trägerfolie (11) als vordere, obere Anlage.

Oberhalb des Messers (55) verläuft der beispielsweise zum Messer (55) parallele obere Schlitz (35). Durch letzteren wird die Deckfolie (12) aus der Sekundärverpackung (30) herausgeführt. Die Schlitzhöhe ist so groß gewählt, dass die Deckfolie (12) und die an ihr haftende Befüllung (1) problemlos hindurch passt.

Beide Schlitze (35, 45) können z.B. mit jeweils mindestens einer Dichtlippe verschlossen sein. Eine ggf. verwendete Dichtlippe kann beispielsweise unter einem spitzen Winkel gegenüber der auszuschiebenden Folie (11, 12) angeordnet sein. Bei einem Winkel unter 45° kann die Folie (11, 12) die Dichtlippe zum Öffnen problemlos wegklappen bzw. wegdrücken.

Um die Befüllung (1) der obersten Folientasche (10) bereitzustellen, betätigt der Nutzer die Transportwalze (61) im Gegenuhrzeigersinn. Die Transportwalze (61) schiebt die Folientasche (10) auf die Schlitze (35, 45) zu, vgl. auch Figur 4. Die Vorderkante (27) der Deckfolie (12) gleitet am Messerrücken (58) entlang durch den Schlitz (35) ins Freie. Bei dieser ersten Anfahrbewegung ist der erforderliche Kraftaufwand sehr niedrig, da im vorderen Bereich der Folientasche (10), also dem Spaltraum (21) nach Figur 1, die Folien (11, 12) unverklebt aufeinander liegen. Erst wenn der vordere Abschnitt (14) des Klebstoffs (13) an der Schneidkante (57) ankommt, muss die Trennarbeit erbracht werden. Dann aber ragen die Folien (11, 12) schon viele Millimeter aus der Sekundärverpackung (30) heraus, vgl. Figuren 4 und 5, so dass die für das Auftrennen der Folien (11, 12) notwendige Kraft auch durch das Herausziehen einer der beiden Folien (11, 12) aufgebracht werden kann. Das Herausziehen erfolgt mit Hilfe der Finger. Die jeweils andere Folie (11) oder (12) wird zwangsweise aus ihrem entsprechenden Schlitz (35) oder (45) so lange herausgeschoben, bis beide Folien (11,12) vollständig getrennt sind. Diese endgültige Trennung findet erst an der hinteren Kante der Folientasche (10) statt, da bis dort hin der beide Folien (11, 12) verbindende Klebstoff (13) reicht.

Bei Bedarf kann zum leichteren Ablösen der Befüllung (1) bzw. des Filmes (1) von der Deckfolie (12) letztere beim Herausziehen nach hinten in Richtung der Transportwalze (61) gezogen werden, vgl. Figur 5. Die Eigensteifigkeit des Films (1) reicht aus, um ein Abheben von der Deckfolie zu ermöglichen. Damit hierbei die Deckfolie (12) nicht einreißt, ist der Übergang zwischen dem oberen Schlitz (35) und dem nach oben weisenden Gehäuseteil des Oberteils (31) abgerundet.

Zum Vermeiden einer Falschbedienung durch Drehen der Transportwalze (61) im Uhrzeigersinn, kann zwischen der Transportwalze (61) und dem Gehäuse (31) eine Verdrehsicherung mittels eines Richtgesperres angeordnet werden.

Um die Trennkraft zum Trennen der beiden Folien (11, 12) zu verringern, kann das Trennwerkzeug (55) auch schräg zur Längsausdehnung der Folientasche (10) angeordnet sein. Ggf. kann auch ein gebogenes, z.B. ein gepfeiltes Messer verwendet werden.

Die Trennwirkung des Trennwerkzeugs (55) muss nicht auf einem Schneidvorgang beruhen. Das Aufbrechen der Verbindung zwischen den Folien (11, 12) kann auch durch ein reines Auseinanderdrücken mittels eines stumpfen Trennwerkzeugs (55) funktionieren. Das entsprechende Trennwerkzeug muss hierzu nur zu Beginn des Trennvorgangs die Folie (12) von der Folie (11) abheben können.

Auch die Schlitze (35, 45) können je nach der Gestaltung des Gehäuses (31, 41) gekrümmt oder gepfeilt ausgeführt sein. Ggf. kann z.B. ein Schlitz (35, 45) auch schräg zur Längsausdehnung der Folientaschen (10) verlaufen.

Selbstverständlich kann der Film (1) innerhalb der Folientasche (10) ggf. auch an der Trägerfolie (11) stärker haften als an der Deckfolie (12). Der Film (1) kann dann aus dem unteren Schlitz (45) entnommen werden. Für diesem Fall müssen eventuell die Schlitze (35, 45) neu dimensioniert werden.

Während dem Herausfördern der Folien (11, 12) aus den Schlitzen (35, 45) wandert in der Sekundärverpackung (30) der Stapel (28) unter der Wirkung der Biege- bzw. Blattfeder (51) nach oben. Die nun oberste Folientasche (10) legt sich an der Transportwalze (61) an. Ein neuer Entnahmevorgang kann eingeleitet werden.

### Bezugszeichenliste:

- 1: folien- oder blattartiges Material, Befüllung Film, Blättchen
- 10: Folientasche, Primärverpackung
- 11: Trägerfolie
- 12: Deckfolie
- 13: Klebstoff
- 14: vorderer Abschnitt von (13)
- 15, 16: Seitenkanten von (11)
- 17: Vorderkante von (11)
- 21: Spaltraum
- 27: Vorderkante von (12)
- 28: Stapel aus (10)
- 30: Sekundärverpackung
- 31: Gehäuseoberteil, Gehäuse
- 32: Absatzbereich, höherliegend
- 33: Stimwand
- 34: Gehäuseöffnung für (61)
- 35: Schlitz für (12) und (1)
- 36, 37: Stege
- 41: Gehäuseunterteil, Gehäuse
- 42: Einspannstelle
- 45: Schlitz für (11)
- 46,47: Stege
- 49: Trennfuge
- 51: Blattfeder
- 52: Andruckplatte
- 53: Arm an (52)
- 54: Schlitz für (51)
- 55: Trennwerkzeug, Messer
- 56: Schneidkante
- 57: Schneidflanke
- 58: Messerrücken
- 61: Transportelement, Transportwalze
- 62: Rotationsachse

## Patentansprüche

1. Vorrichtung zum Aufbewahren und Bereitstellen von gestapelten mit folien- oder blattartigen Materialien (1) befüllten Folientaschen (10),
- wobei die Folientaschen (10) aus mindestens einer Trägerfolie (11) und mindestens einer Deckfolie (12) bestehen,
- wobei mindestens die flächenmäßig größere Folie (11) zwei parallele Seitenkanten (15, 16) aufweist,
- wobei zwischen der Trägerfolie (11) und der Deckfolie (12) das folien- oder blattartige Material (1) gas- und feuchtedicht eingeschlossen ist,
- wobei die Trägerfolie (11) an mindestens einer quer oder schräg zur Seitenkante (15, 16) liegenden Kante (17) über die nächstliegende Kante (27) der Deckfolie (12) übersteht,
- wobei die Vorrichtung ein mindestens einfach geteiltes Gehäuse (31, 41) umfasst, an dessen seitlichen Innenseiten die Seitenkanten (15, 16) der gestapelten Folientaschen (10) anliegen, während an einer vorderen Innenseite diejenige Kante (17) der Trägerfolie (11) anliegt, die über die Kante (27) der dazugehörenden Deckfolie (12) übersteht,
- wobei die gestapelten Folientaschen (10) federbelastet gegen die obere Innenseite des Gehäuses (31, 41) gepresst werden,
**dadurch gekennzeichnet,**
- **dass** der vordere, obere Bereich des Gehäuses (31, 41) zwei Schlitze (35, 45) für das getrennte Auswerfen der einzelnen Folientaschenteile (11, 12) hat, wobei das folien- oder blattartige Material (1) mit einem der Folientaschenteile (11) oder (12) ausgeworfen wird,
- **dass** zwischen den Schlitzen (35) und (45) ein Trennwerkzeug (55) zum Trennen von Träger- (11) und Deckfolie (12) angeordnet ist, an dem die oberste Trägerfolie (11) räumlich vor der Vorderkante (27) der Deckfolie (12) anliegt und
- **dass** oberhalb der vorderen Hälfte der obersten Folientasche (10) - auf dieser aufliegend - ein um eine Rotationsachse (62) rotierbares Transportelement (61) zumindest annähernd quer zu den Seitenkanten (15, 16) in einer Gehäuseöffnung (34) angeordnet ist.

2. Vorrichtung gemäß Anspruch 1, **dadurch gekennzeichnet, dass** zwischen dem vordersten Punkt der Vorderkante (27) und der nächsten Klebe- oder Versiegelungsstelle (14) zwischen der Trägerfolie (11) und der Deckfolie (12) keine Haftung besteht.

3. Vorrichtung gemäß Anspruch 1, **dadurch gekennzeichnet, dass** die Trägerfolie (11) mit der Deckfolie (12) verklebt ist.

4. Vorrichtung gemäß Anspruch 1, **dadurch gekennzeichnet, dass** die Vorderkante (27) der Deckfolie (12) eine halbelliptische Kontur hat.

5. Vorrichtung gemäß Anspruch 1, **dadurch gekennzeichnet, dass** das Transportelement eine zylindrische Transportwalze (61) ist.

6. Vorrichtung gemäß Anspruch 5, **dadurch gekennzeichnet, dass** die Transportwalze (61) zumindest eine gummiartige Beschichtung aufweist.

7. Vorrichtung gemäß Anspruch 1, **dadurch gekennzeichnet, dass** der obere Schlitz (35), durch den die Deckfolie (12) ausgeworfen wird, zwischen der Transportwalze (61) und dem unteren Schlitz (45) angeordnet ist.

8. Vorrichtung gemäß Anspruch 1, **dadurch gekennzeichnet, dass** das einzelne folien- oder blattartige Material (1) eine einen pharmazeutischen Wirkstoff enthaltende Arzneiform ist.

9. Vorrichtung gemäß Anspruch 1, **dadurch gekennzeichnet, dass** mindestens die flächenmäßig größere Folie (11) abschnittsweise zwei parallele Seitenkanten (15, 16) aufweist.

10. Vorrichtung gemäß Anspruch 1, **dadurch gekennzeichnet, dass** das Trennwerkzeug zum Trennen von Trägerfolie (11) und Deckfolie (12) ein Messer (55) ist, das einen Winkel von ca. 30° zur Oberfläche der obersten horizontalen Folientasche (10) aufweist und plan an der Oberfläche der obersten Trägerfolie (11) anliegt.

11. Vorrichtung gemäß Anspruch 1, **dadurch gekennzeichnet, dass** das Gehäuse ein wannenförmiges Unterteil (41) und ein deckelartiges Oberteil (31) umfasst, wobei das Unterteil (41) die gestapelten Folientaschen (10) aufnimmt und das Oberteil die beiden Schlitze (35, 45) und die Transportwalze (61) trägt.

12. Vorrichtung gemäß Anspruch 1, **dadurch gekennzeichnet, dass** das folien- oder blattartige Material (1) an der Deckfolie (12) stärker haftet als an der Trägerfolie (11).

## Claims

1. A device for storing and providing stacked film pockets (10) filled with film-type or laminar materials (1),
- in which the film pockets (10) consist of at least one support film (11) and at least one cover film (12),
- in which at least the film (11) of greater surface area has two parallel side edges (15, 16),
- in which the film-type or laminar material (1) is enclosed in a gas-tight and moisture-tight manner between the support film (11) and the cover film (12),
- in which the support film (11), at at least one edge (17) lying transversely or obliquely with respect to the side edge (15, 16), protrudes beyond the nearest edge (27) of the cover film (12),
- in which the device comprises a housing (31, 41) which is partitioned at least once and on whose lateral inner faces the side edges (15, 16) of the stacked film pockets (10) bear, while the edge (17) of the support film (11) protruding beyond the edge (27) of the associated cover film (12) bears on a front inner face,
- in which the stacked film pockets (10) are pressed with spring-loading against the upper inner face of the housing (31, 41),
**characterized in that**
- the front, upper area of the housing (31, 41) has two slits (35, 45) for separate ejection of the individual film pocket parts (11, 12), the film-type or laminar material (1) being ejected with one of the film pocket parts (11) or (12),
- a separating tool (55) for separating support film (11) and cover film (12) is arranged between the slits (35) and (45), on which separating tool (55) the uppermost support film (11) bears spatially in front of the front edge (27) of the cover film (12), and
- above the front half of the uppermost film pocket (10), and bearing thereon, a transport element (61) which is rotatable about an axis of rotation (62) and is at least approximately transverse with respect to the side edges (15, 16) is arranged in a housing opening (34).

2. The device as claimed in claim 1, **characterized in that**, between the frontmost point of the front edge (27) and the nearest adhesion point or sealing point (14), there is no adherence between the support film (11) and the cover film (12).

3. The device as claimed in claim 1, **characterized in that** the support film (11) is adhesively bonded to the cover film (12).

4. The device as claimed in claim 1, **characterized in that** the front edge (27) of the cover film (12) has a semi-elliptical contour.

5. The device as claimed in claim 1, **characterized in that** the transport element is a cylindrical transport roller (61).

6. The device as claimed in claim 5, **characterized in that** the transport roller (61) has at least a rubber-like coating.

7. The device as claimed in claim 1, **characterized in that** the upper slit (35), through which the cover film (12) is ejected, is arranged between the transport roller (61) and the lower slit (45).

8. The device as claimed in claim 1, **characterized in that** the individual film-type or laminar material (1) is a drug form containing a pharmaceutical active substance.

9. The device as claimed in claim 1, **characterized in that** at least the film (11) of greater surface area has two parallel side edges (15, 16) in some sections.

10. The device as claimed in claim 1, **characterized in that** the separating tool for separating support film (11) and cover film (12) is a blade (55) which is at an angle of ca. 30° to the surface of the uppermost horizontal film pocket (10) and bears flat on the surface of the uppermost support film (11).

11. The device as claimed in claim 1, **characterized in that** the housing has a trough-shaped bottom part (41) and a top part (31) like a lid, the bottom part (41) receiving the stacked film pockets (10), and the top part having the two slits (35, 45) and the transport roller (61).

12. The device as claimed in claim 1, **characterized in that** the film-type or laminar material (1) adheres more strongly to the cover film (12) than it does to the support film (11).

## Revendications

1. Dispositif de conservation et de mise à disposition de pochettes en film empilées (10) remplies de matériaux du type feuille ou page (1),
- les pochettes en film (10) étant constituées d'au moins un film de support (11) et d'au moins un film de recouvrement (12),
- au moins le film de plus grande surface (11) comprenant deux arêtes latérales (15, 16) parallèles,
- le matériau du type feuille ou page (1) étant enfermé de manière étanche au gaz et à l'humidité entre le film de support (11) et le film de recouvrement (12),
- le film de support (11), au niveau au moins d'une arête (17) située de façon transversale ou oblique à l'arête latérale (15, 16), dépassant de l'arête suivante (27) du film de recouvrement (12),
- le dispositif comportant un logement (31, 41) divisé au moins une fois, contre les côtés intérieurs latéraux duquel reposent les arêtes latérales (15, 16) des pochettes en film (10) empilées, tandis que l'arête (17) du film de support (11) repose contre un côté intérieur avant, laquelle arête dépasse de l'arête (27) du film de recouvrement (12) en faisant partie,
- les pochettes en film (10) empilées étant élastiquement pressées contre le côté intérieur supérieur du logement (31, 41),
**caractérisé en ce que**
- la zone supérieure avant du logement (31, 41) présente deux fentes (35, 45) pour éjecter séparément les parties individuelles (11, 12) de la pochette en film, le matériau du type feuille ou page (1) étant éjecté avec une des parties (11) ou (12) de la pochette en film,
- **en ce qu'**un outil de séparation (55) destiné à séparer le film de support (11) et le film de recouvrement (12) est disposé entre les fentes (35) et (45), outil contre lequel le film de support le plus supérieur (11) repose dans l'espace devant l'arête avant (27) du film de recouvrement (12) et
- **en ce qu'**au-dessus de la moitié avant de la pochette en film la plus supérieure (10), un élément transporteur (61) - reposant sur celle-ci - rotatif autour d'un axe de rotation (62), est disposé dans un orifice de logement (34) au moins approximativement transversalement aux arêtes latérales (15, 16).

2. Dispositif selon la revendication 1, **caractérisé en ce qu'**il n'existe aucune adhérence entre le point le plus avant de l'arête avant (27) et le point de collage ou de scellement (14) suivant entre le film de support (11) et le film de recouvrement (12).

3. Dispositif selon la revendication 1, **caractérisé en ce que** le film de support (11) est collé au film de recouvrement (12).

4. Dispositif selon la revendication 1, **caractérisé en ce que** l'arête avant (27) du film de recouvrement (12) présente un contour semi-elliptique.

5. Dispositif selon la revendication 1, **caractérisé en ce que** l'élément de transport est un rouleau transporteur cylindrique (61).

6. Dispositif selon la revendication 5, **caractérisé en ce que** le rouleau transporteur (61) comprend au moins un revêtement caoutchouteux.

7. Dispositif selon la revendication 1, **caractérisé en ce que** la fente supérieure (35), à travers laquelle est éjecté le film de recouvrement (12), est disposée entre le rouleau transporteur (61) et la fente inférieure (45).

8. Dispositif selon la revendication 1, **caractérisé en ce que** le matériau du type feuille (1) individuel est une forme médicamenteuse contenant une substance pharmaceutique.

9. Dispositif selon la revendication 1, **caractérisé en ce qu'**au moins le film de plus grande surface (11) comprend par section deux arêtes latérales (15, 16) parallèles.

10. Dispositif selon la revendication 1, **caractérisé en ce que** l'outil de séparation destiné à séparer le film de support (11) et le film de recouvrement (12) est une lame (55), qui limite un angle d'approximativement 30 degrés par rapport à la surface de la pochette en film horizontale la plus supérieure (10) et repose à plat contre la surface du film de support le plus supérieur (11).

11. Dispositif selon la revendication 1, **caractérisé en ce que** le logement comporte une partie inférieure en cuvette (41) et une partie supérieure du type couvercle (31), la partie inférieure (41) recevant les pochettes en film (10) empilées et la partie supérieure supportant les deux fentes (35, 45) et le rouleau transporteur (61).

12. Dispositif selon la revendication 1, **caractérisé en ce que** le matériau de type feuille ou page (1) adhère plus fortement au film de recouvrement (12) qu'au film de support (11).
